# EUROPEAN PATENT SPECIFICATION

(11) **EP 1 385 450 B1**
(45) Date of publication and mention of the grant of the patent: **14.03.2007**
(21) Application number: 02704179.7
(22) Date of filing: 18.01.2002
(51) Int. Cl.: A61F 2/06, A61M 25/00

(54) **CATHETER SYSTEM WITH SPACER MEMBER**
KATHETERSYSTEM MIT DISTANZTEIL
SYSTEME DE CATHETER AVEC ELEMENT DE PIECE D'ESPACEMENT

(30) Priority: 18.01.2001 US 765719; 17.09.2001 US 954763
(43) Date of publication of application: 04.02.2004
(73) Proprietor: ev3 Peripheral, Inc., Plymouth, MN 55442 (US)
(72) Inventor: THOMPSON, Paul, J., Minnetonka, MN 55343 (US); GUNDERSON, Richard, C., Maple Grove, MN 55311 (US)
(74) Representative: Prins, Adrianus Willem
(86) International application number: PCT/US2002/001594
(87) International publication number: WO 2002/056953

(56) References cited:
- EP-A- 0 788 802
- WO-A-99/47071
- US-A- 5 899 917

## Description

### BACKGROUND OF THE INVENTION

### 1. Field of Invention

This invention pertains to a system for delivering a stent to a site in a body lumen. More particularly, this invention pertains to a stent delivery system with *i*mproved structure between tubular members. A system according to the preamble of claim 1 is disclosed in EP-A- 0 839 504.

### 2. Description of the Prior Art

Stents are widely used for supporting a lumen structure in a patient's body. For example, stents may be used to maintain patency of a coronary artery, other blood vessel or other body lumen.

Commonly, stents are metal, tubular structures. Typically stents have an open-cell structure. Stents are passed through the body lumen in a collapsed state. At the point of an obstruction or other deployment site in the body lumen, the stent is expanded to an expanded diameter to support the lumen at the deployment site.

In certain designs, stents are expanded by balloon dilation at the deployment site. These stents are typically referred to as "balloon expandable" stents. Other stents are so-called "self-expanding" stents that enlarge at a deployment site by inherent elasticity or shape-memory characteristics of the stents. Frequently self-expanding stents are made of a super-elastic material such as a nickel-titanium alloy (i.e., nitinol).

A delivery technique for stents is to mount the collapsed stent on a distal end of a stent delivery system. Such a system would include an outer tubular member and an inner tubular member. Prior to advancing the stent delivery system through the body lumen, a guide wire is first passed through the body lumen to the deployment site. The inner tube of the delivery system is hollow throughout its length such that it can be advanced over the guide wire to the deployment site.

The combined structure (i.e., stent mounted on stent delivery system) is passed through the patient's lumen until the distal end of the delivery system arrives at the deployment site within the body lumen. The deployment system may include radio-opaque markers to permit a physician to visualize positioning of the stent under fluoroscopy prior to deployment.

At the deployment site, the outer sheath is retracted to expose a self-expanding stent, or fluid is injected to inflate a balloon which expands a balloon-expandable tube stent. Following expansion of the stent, the delivery system can be removed through the body lumen leaving the stent in place at the deployment site.

Prior art stent delivery systems use inner and outer tubes of generally uniform diameters and circular cross-section throughout their length. This design relies upon the dynamics of fluid flow to retain spacing between the tubes.

In the event the outer diameter of the inner prior art tube is substantially less than the inner diameter of the outer prior art tube, the inner tube could bend relative to the outer tube such that surfaces of the inner tube abut surfaces of the outer tube. As a result, axial forces applied to advance the stent delivery system could be stored in the bent inner tube. Such energy could be suddenly released with sudden and undesired rapid advance or retraction of the distal tip of the tubes when the inner tube straightens.

The likelihood of this sudden jumping phenomenon could be reduced by having the inner and outer tube diameters be as close as possible. However, such close tolerances result in a very small annular gap between the inner and outer tubes which results in increased resistance to fluid flow between the inner and outer tube.

### SUMMARY OF THE INVENTION

A catheter system for use in a body lumen of a patient is disclosed. One aspect of the present invention relates to the catheter system having a spacer member. In certain embodiments, the catheter system can be adapted to deploy a self-expanding stent or a balloon-expandable stent. Another aspect of the present invention relates to a stent delivery system including an arrangement for allowing fluid exchange with a patient.

### BRIEF DESCRIPTION OF THE DRAWINGS

Fig. 1 is a side elevation view of one embodiment of a stent delivery system according to the present invention.
Fig. 2 is a side sectional view of a distal end of the stent delivery system of Fig. 1, shown in Fig. 1 as Detail A.
Fig. 3 is a side sectional view of a proximal end of the stent delivery system of Figure 1, shown in Fig. 1 as Detail B.
Fig. 4 is a sectional view of a second handle of the stent delivery system of Fig. 1 and showing, in section, a guide wire port, shown in Fig. 1 as Detail C.
Fig. 5 is a cross-sectional view of the inner and outer tubular members of the stent delivery system of Fig. 1 taken along lines 5-5 of Fig. 3 and showing a first embodiment of a spacer configuration.
Fig. 6 is a perspective view of one-half of a handle of the stent delivery system of Fig. 1 with the opposite half being of identical construction.
Fig. 7A is a perspective view of one of the handles of the stent delivery system of Fig. 1.
Fig. 7B is a front end view of the handle of Fig. 7A.
Fig. 7C is a back end view of the handle of Fig. 7A.
Fig. 7D is a front side view of the handle of Fig. 7A.
Fig. 7E is a back side view of the handle of Fig. 7A.
Fig. 7F is a top view of the handle of Fig. 7A.
Fig. 7G is a bottom view of the handle of Fig. 7A.
Fig. 8 is a side view of another embodiment of the stent delivery system according to the present invention showing a cross section of the manifold and stent deployment arrangement.
Fig. 9 is an enlarged detail view of the manifold of Fig. 8.
Fig. 10 is an enlarged detail view of Fig. 8 taken at Detail B.
Fig. 11 is a sectional view of Fig. 8 taken along line 11-11.
Fig. 12 is a sectional view of Fig. 8 taken along line 12-12 and showing a second embodiment of a spacer configuration.
Fig. 13 is a sectional view of Fig. 8 taken along line 13-13.
Fig. 14 is a sectional view of Fig. 10 taken along line 14-14.
Fig. 15 is a cross section view of a third embodiment of a spacer configuration suitable for use with a delivery system in accordance with the principles of the present invention.
Fig. 16 is a cross section view of a fourth embodiment of a spacer configuration suitable for use with a delivery system in accordance with the principles of the present invention.
Fig. 17 is a cross section view of a fifth embodiment of a spacer configuration suitable for use with a delivery system in accordance with the principles of the present invention.
Fig. 18 is a cross section view of a sixth embodiment of a spacer configuration suitable for use with a delivery system in accordance with the principles of the present invention.
Fig. 19 is a cross section view of a seventh embodiment of a spacer configuration suitable for use with a delivery system in accordance with the principles of the present invention.
Fig. 20 is a cross section view of an eighth embodiment of a spacer configuration suitable for use with a delivery system in accordance with the principles of the present invention.
Fig. 21 is a cross section view of a ninth embodiment of a spacer configuration suitable for use with a delivery system in accordance with the principles of the present invention.
Fig. 22 is a cross section view of a tenth embodiment of a spacer configuration suitable for use with a delivery system in accordance with the principles of the present invention.
Fig. 23 is a top perspective view showing an eleventh spacer configuration in accordance with the principles of the present invention.
Fig. 24 is a cross section view of the spacer configuration of Fig. 23.

### DETAILED DESCRIPTION

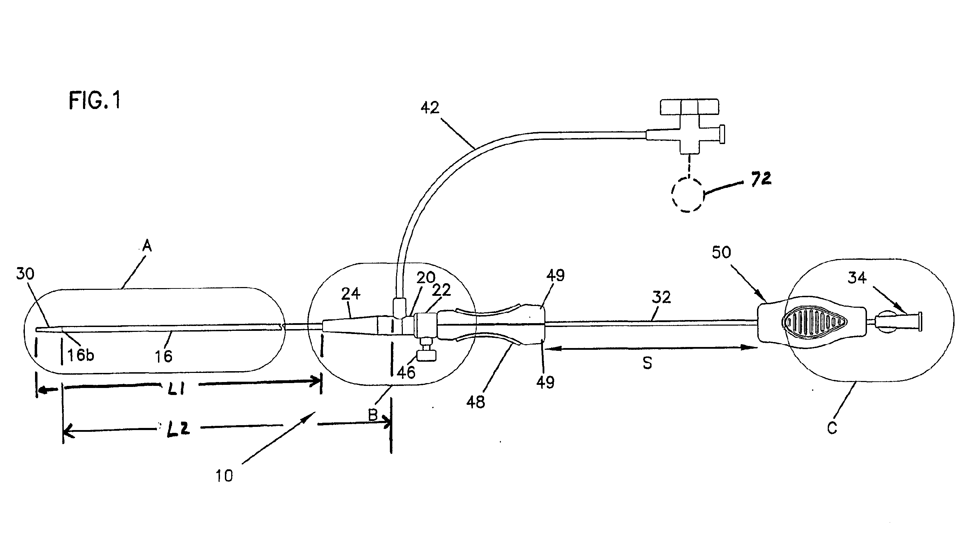

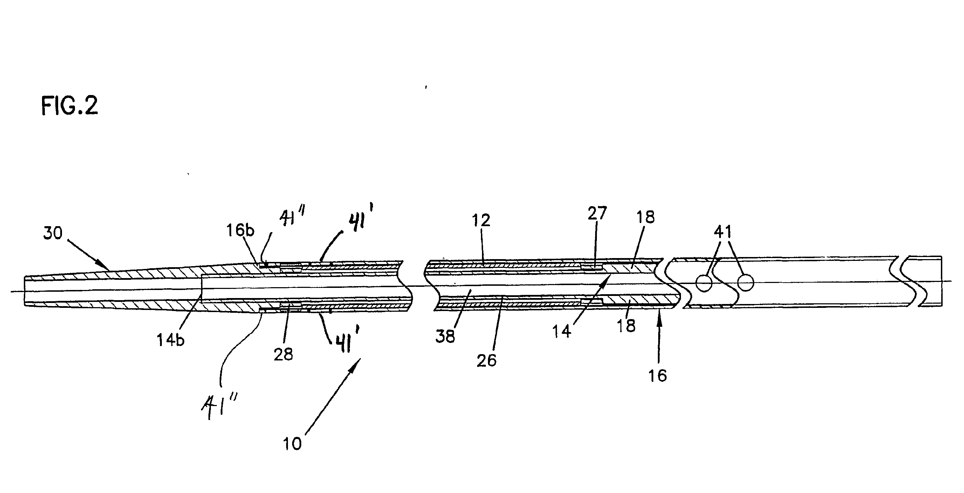

With initial references to Figs. 1 - 4, a first embodiment of a stent delivery system 10 is shown. The stent delivery system 10 is for delivery of a stent 12 (schematically shown only in Fig. 2) to a deployment site in a body lumen of a patient's body. By way of non-limiting, representative example, the stent 12 may be a self-expanding, open-celled, tubular stent having a construction such as that shown in U.S. Pat. No. 6,132,461 and formed of a self-expanding, shape-memory or superelastic metal such as nitinol, or the like. The stent 12 may also be a coil stent or any other self-expanding stent.

The stent 12 is carried on the stent delivery system 10 in a collapsed (or reduced diameter) state. Upon release of the stent 12 from the stent delivery system 10 (as will be described), the stent 12 expands to an enlarged diameter to abut against the walls of the patient's lumen in order to support patency of the lumen.

The lumen of a patient may include, for example, any vascular lumen or duct, as well as other lumens or ducts including biliary, esphageal, bronchial, urethral, or colonic lumens or ducts. It is contemplated that the catheter system disclosed may be sized accordingly to the lumen or duct to which it applies.

The stent delivery system 10 includes an inner tubular member 14 and an outer tubular member 16. Both of the inner and outer tubular members 14 and 16 extend from proximal ends 14a, 16a to distal ends 14b, 16b.

The outer tubular member 16 is sized to be axially advanced through the patient's body lumen for the distal end 16b to be placed near the deployment site in the body lumen and with the proximal end 16a remaining external to the patient's body for manipulation by an operator. By way of example, the outer tubular member 16 (also referred to as a sheath) may be a braid-reinforced polyester of tubular construction to assist in resisting kinks and to transmit axial forces along the length of the sheath 16. The outer tubular member 16 may be of widely varying construction to permit varying degrees of flexibility of the outer tubular member 16 along its length.

The proximal end 16a of the outer tubular member 16 is bonded to a manifold housing 20. The manifold housing 20 is threadedly connected to a lock housing 22. A strain relief jacket 24 is connected to the manifold housing 20 and surrounds the outer tubular member 16 to provide strain relief for the outer tubular member 16.

The outer tubular member 16 defines a usable or operating length L1 of the stent delivery system. The operating length L1 includes a portion of the stent delivery system that is inserted into a patient's lumen. The operating length L1 extends from the strain relief jacket 24 to the end of a distal tip member 30, as shown in Fig. 1. The operating length may comprise a variety of lengths including, for example, 60 cm, 80 cm, 120 cm, 135 cm, and 150 cm.

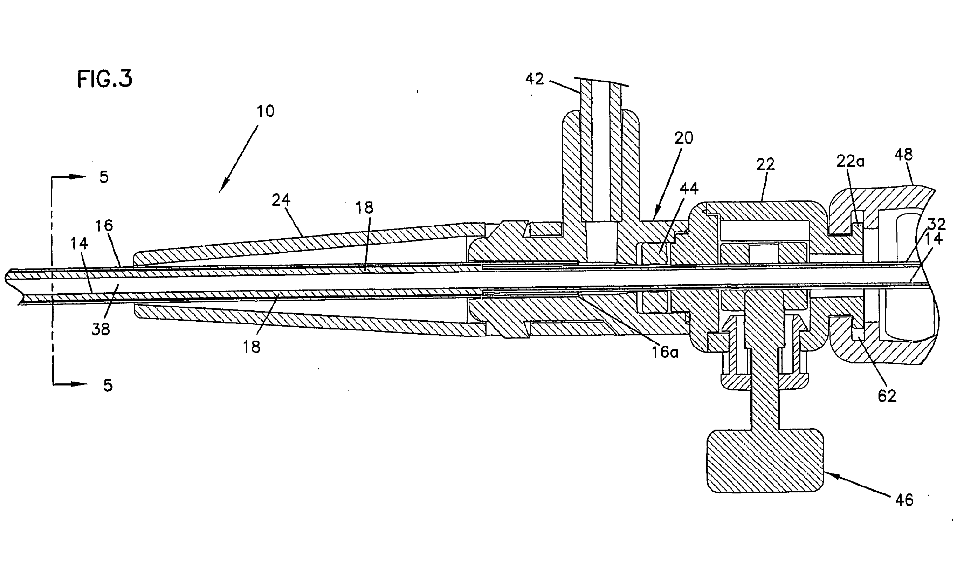

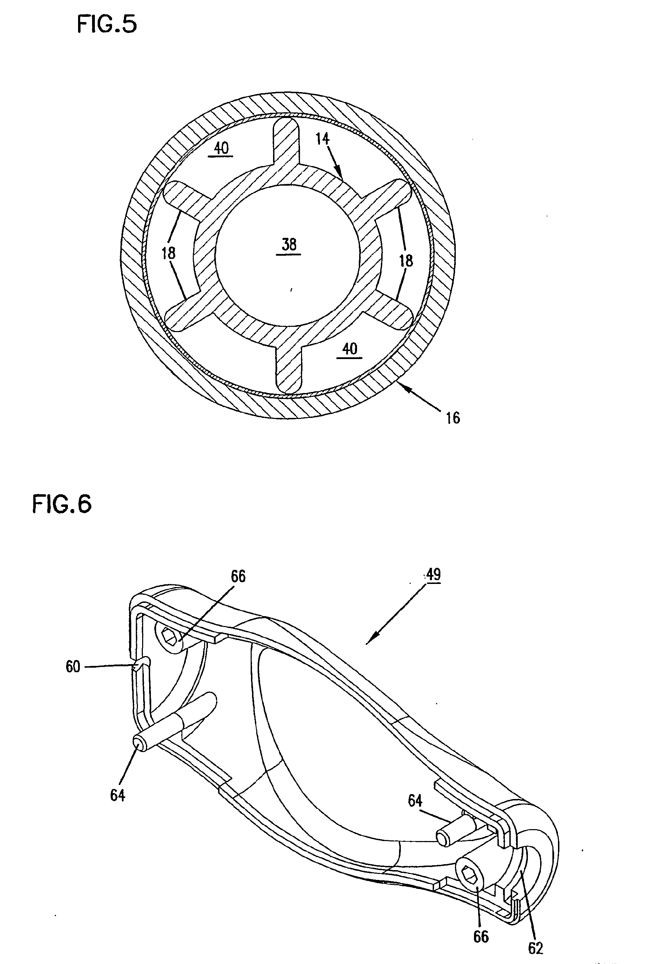

The inner tubular member 14 is preferably formed of nylon but may be constructed of any suitable material. Along a portion of its length from the proximal end 16a of the outer tubular member 16 to a stent attachment location 26 (shown in Fig. 2), the inner tubular member 14 is a cylinder with a spacer member 18 which, in one embodiment, comprises radially projecting and axially extending splines (shown with reference to Figs. 3 and 5). The function and purpose of the spacer member 18 will be described later.

At the distal end 14b of the inner tubular member 14, the inner tubular member 14 has no splines. The splineless length of the distal end of the inner tubular member 14 is of sufficient length to be greater than an axial length of the stent 12. This distal splineless length of the inner tubular member defines the stent attachment location 26 between spaced apart radio-opaque markers 27, 28 which are attached to the inner tubular member 14. The radio-opaque markers 27, 28 permit a physician to accurately determine the position of the stent attachment location 26 within the patient's lumen under fluoroscopy visualization. The distal tip member 30 is secured to the reduced and splineless portion of the inner tubular member 14. The distal tip member 30 is tapered and highly flexible to permit advancement of the stent deployment system 10 through the patient's lumen and minimize trauma to the walls of the patient's lumen.

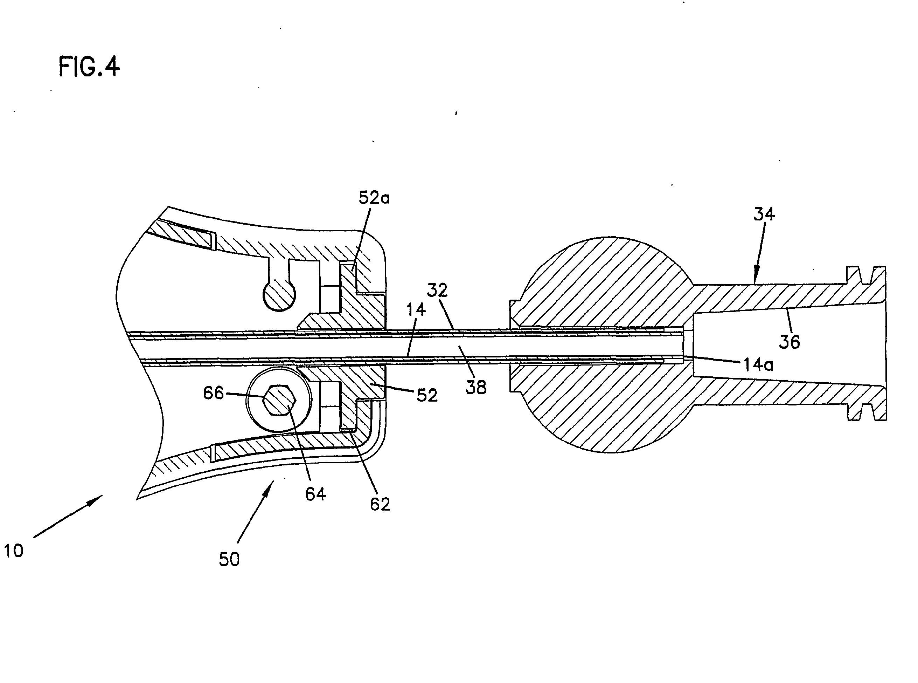

In the first embodiment shown in Figs. 3 and 4, from the proximal end 16a of the outer tube 16 to the inner tube proximal end 14a, the inner tube 14 is cylindrical and splineless. The inner tube 14 passes through both the manifold housing 20 and lock housing 22. A stainless steel jacket 32 surrounds and is bonded to the inner tubular member 14 from the proximal end 14a up to and abutting the splines 18.

At the inner tube proximal end 14a, a port housing 34 is bonded to the stainless steel jacket 32. The port housing 34 has a tapered bore 36 aligned with an inner lumen 38 of the tubular member 14. The inner lumen 38 extends completely through the inner tubular member 14 so that the entire delivery system 10 can be passed over a guide wire (not shown) initially positioned within the patient's lumen. Opposing surfaces of the inner and outer tubular members 14 and 16, define a passageway, fluid channel, or first lumen 40 (best seen in Figs. 5 and 11-22). The first lumen 40 thereby is defined by the inner diameter of outer tubular member 16 and the outer diameter of the inner tubular member 14. Depending upon the diameter of the catheter, the first lumen 40 may have a radial distance between the opposing surfaces of inner and outer tubular members of about .003 inches to .2 inches, inclusively, for example.

The first lumen 40 defines a first lumen or fluid channel length L2, shown generally in Fig. 1. The fluid channel length L2 extends from the proximal end of the outer tubular member 16a, shown in Fig. 3, to the distal end of the outer tubular member 16b, shown in Fig 2. The spacer member 18 traverses along a predetermined percentage of the fluid channel length L2. The predetermined percentage may be at least 10%, at least 25%, at least 50%, or at least 75% of the fluid channel length L2. Preferably, the predetermined percentage over which the spacer member 18 traverses the fluid channel length L2 is at least 90%. Similarly, the spacer member 18 may traverse along a predetermined percentage of the operating length L1.

By reason of the spacer member 18, the inner tubular member 14, cannot bend relative to the outer tubular member 16, thereby avoiding the problems associated with the prior art designs as previously discussed. Also, since the splines 18 contact the outer tubular member only at small surface areas along the length, reduced friction results from sliding motion between the inner and outer tubular members 14, 16, of self-expanding stent delivery systems.

Referring to Figs. 1 and 3, the manifold housing 20 of the first embodiment carries an admission port 42 for injecting a contrast media or other fluid such as Saline, Nitroglycerine, or other therapeutic agents, into the interior of the manifold housing 20. The interior of the manifold housing 20 is in fluid flow communication with the first lumen 40. Discharge ports (i.e. fluid exchange ports for discharging or extracting fluid) 41, 41' (shown in Fig. 2) are formed through the outer tubular member 16 to permit contrast media, for example, to flow from the first lumen 40 into the patient's body lumen. It is to be understood one or more discharge ports may be formed through the outer tubular member. For example, multiple discharge ports may be formed in the outer tubular member to permit greater flow of the contrast media into the patient's body lumen. The contrast media discharged through the discharge ports aids the user in determining the characteristics of the flow through the patient's lumen.

The discharge ports 41 and 41' are formed in a portion of the outer tubular member proximate the stent attachment location 26 (i.e. the sheath which covers the stent). An arrangement providing only discharge ports 41 without oppositely positioned discharge ports 41' or only discharge ports 41' without oppositely positioned discharge ports 41 is contemplated. Alternatively, discharge ports 41" in the form of end notches formed at a distal most end of the outer tube 16 can be used. The discharge openings 41' and 41" are preferably located distally with respect to a longitudinal mid-point of the stent 12. Most preferably, openings 41' and 41" are located adjacent to or distal to the distal end of the stent 12.

In use, the discharge ports 41, 41' provide several advantages. One advantage of the oppositely positioned discharge ports is that when intending to use a contrast media for flow analysis, for example, the user may advance the stent delivery system 10 in a direction either with the direction of flow within the patient's lumen or against the direction of flow within the patient's lumen. To illustrate, if the user advances the system in a direction with the flow in the patient's lumen, contrast media discharged from discharge ports 41 will enter the patient's fluid stream and the user may observe the flow of the contrast media through the desired deployment location or area of blockage. However, the contrast media discharged from discharge ports 41' is down stream from the blockage area and does not flow through the desired deployment location or area of blockage. In the alternative, if the system is advanced within the patient's lumen in a direction against the flow, contrast media from discharge ports 41' flows through the desired deployment location. In an arrangement including only discharge ports 41, for example, the user advances the delivery system in a direction corresponding to the patient's lumen flow.

Another advantage provided by the discharge ports 41, 41' involves obtaining information related to fluid pressure differentials within the patient's lumen. The stent delivery system 10 may include a pressure measurement device 72 (shown in phantom in Fig. 1) that provides a measurement of the fluid pressure within the patient's lumen by measuring the fluid pressure within the fluid channel 40, which equalizes to the patient's lumen fluid pressure via communication through the discharge ports. To illustrate, prior to deployment, fluid pressure transmits through the fluid channel 40 providing a first pressure reading. As the stent is expanded, fluid in the patient's lumen begins to flow and the pressure decreases. Correspondingly, the pressure in the fluid channel decreases permitting the user to monitor the pressure differential in the patient's lumen.

The user may also monitor lumen flow through a deployed stent by measuring the pressure prior to the blockage and subsequent to the blockage. To illustrate, after stent deployment, a first pressure reading may be taken wherein the discharge ports of the outer tubular member are in a retracted position within an area prior to the blockage, for example. A second pressure reading may then be obtained subsequent to the area of blockage by axially sliding the outer tubular member into its original protracted position and through the expanded stent, wherein the discharge ports are located prior to the blockage.

It is further contemplated that simultaneous pressure readings, one in an area prior to the blockage and another in an area subsequent to the blockage, may be provided by an arrangement incorporating a first fluid channel and a second fluid channel (not shown). The first and second fluid channels or lumens would correspond to respective first and second discharge apertures where, for example, the first discharge apertures are located prior to the stent attachment location and are in fluid communication with the first fluid channel, and the second discharge apertures are located subsequent to the stent attachment location and are in fluid communication with the second fluid channel. A pressure measurement device monitoring the different pressures within the first fluid channel and the second fluid channel would provide simultaneous pressure readings.

In an alternative embodiment, a self-expanding stent delivery system having a fluid channel between inner and outer members and including one or more discharge ports, may or may not include a spacer member.

Referring again now to Fig. 3, an O-ring 44 surrounds the stainless steel jacket 32 between the manifold housing 20 and lock housing 22. Upon threaded connection of the manifold housing 20 to the lock housing 22, the O-ring 44 compresses against the stainless steel jacket 32 in sealing engagement to prevent contrast media from flowing in any path other than through the first lumen 40.

The lock housing 22 carries a threaded locking member (or lock nut) 46 which can be turned to abut the stainless steel jacket 32. The lock nut 46 can be released to free the stainless steel jacket to move axially. According, when the lock nut 46 engages the jacket 32, the jacket 32 (and attached inner tubular member 14) cannot move relative to the lock housing 22, manifold housing 20 or the outer tubular member 18. Upon release of the lock nut 46, the inner tubular member 14 and outer tubular member 18 are free to slide axially relative to one another between a transport position and a deploy position.

As best shown in Fig. 1, first and second handles 48, 50 are secured to the lock housing 22 and jacket 32, respectively. In the transport position, the handles 48, 50 are spaced apart and the outer tubular member 16 covers the stent attachment location 26 to prevent premature deployment of the stent 12. When the handle 48 is pulled rearwardly toward the handle 50, the outer tubular member 16 slides rearwardly or proximally relative to the inner tubular member 14. Preferably, the outer tubular member 16 slides rearwardly a distance sufficient to fully expose the stent attachment location 26 and permit the stent 12 to freely expand toward its fully expanded diameter. After such expansion, the stent delivery system can be proximally withdrawn through the expanded stent and removed.

The first handle 48 is rotatably mounted on a flange 22a (as shown in Fig. 3) of the lock housing 22. The first handle 48 surrounds the stainless steel jacket 32 and is freely rotatable about the longitudinal axis of the jacket 32 and freely rotatable about the flange 22a. The first handle 48 is axially affixed to the lock housing 22 such that axial forces applied to the first handle 48 are transmitted through the lock housing 22 and manifold housing 20 to the outer tubular member 16 to axially move the outer tubular 16. However, rotary action of the first handle 48 about the axis of the stainless steel jacket 32 is not transmitted to the housings 20, 22 or to the outer tubular member 16 by reason of the free rotation of the first handle 48 on flange 22a.

The second handle 50 is mounted on an anchor 52 (shown in Fig. 4) which is bonded to the stainless steel jacket 32 through any suitable means (such as by use of adhesives). The anchor 52 includes a flange 52a which is radial to the axis of the stainless steel jacket 32. The second handle 50 is mounted on the flange 52a and is free to rotate on the anchor 52 about the axis of the stainless steel jacket 32. However, axial forces applied to the handle 50 are transmitted to the stainless steel jacket 32 which, being bonded to the inner tubular member 14, results in axial movement of the inner tubular member 14.

With the handle construction described above, relative axial movement between the handles 48, 50 results in relative axial movement between the inner and outer tubular members 14, 16. Rotational movement of either of the handles 48, 50 does not affect rotational positioning of the inner or outer tubular members 14, 16 and does not affect axial positioning of the inner and outer tubes 14, 16.

The free rotation of the handles 48, 50 results in ease of use for a physician who may position his or her hands as desired without fear of interfering with any axial positioning of the inner and outer tubular members 14, 16. The spacing between the handles 48, 50 is equal to the stroke between the transport position and the deploy position of the tubular members 14, 16. As a result, the spacing permits the operator to have ready visual indication of the relative axial positioning between the inner and outer tubular members 14, 16. This relative axial positioning can be fixed by engaging the lock nut 46. In any such positioning, contrast media can be injected through the admission port 42 into the chamber 40 with the contrast media flowing out of the side ports 41 into the body lumen to permit visualization under fluoroscopy.

With reference to Fig. 6, each of the handles 48, 50 is formed of identical halves 49 (Fig. 6) of injected molded plastic to permit ease of manufacture. When the handle halves 49 are joined together, pins 64 are received in aligned openings 66 of an opposing half 49 for attachment and permanent connection of two halves 49. The halves 49 include first openings 60 proximate to the outer diameter of the stainless steel jacket 32. At opposite ends, the halves 49 include annular recesses 62 to receive either of flanges 22a or 52a for rotatable attachment upon joinder of two halves 49.

With stent deployment systems having premounted stents of various axial lengths, the positioning of the second handle 50 on the stainless steel jacket 32 can be selected at time of assembly so that a spacing S (see Fig. 1) between the handles 48, 50 corresponds to the length of the stent 12 carried on the stent deployment system. For example, in the first embodiment, the spacing S is preferably about 10 millimeters longer than the deployed length of the stent. Accordingly, the user will know that the outer tubular member 16 has been fully retracted when the handles 48, 50 have been pushed completely together to completely release the stent 12. Also, the freely rotatable handles 48, 50 are easy to hold from any angle without slippage. The lock nut 46 ensures that the stent 12 will not deploy prematurely.

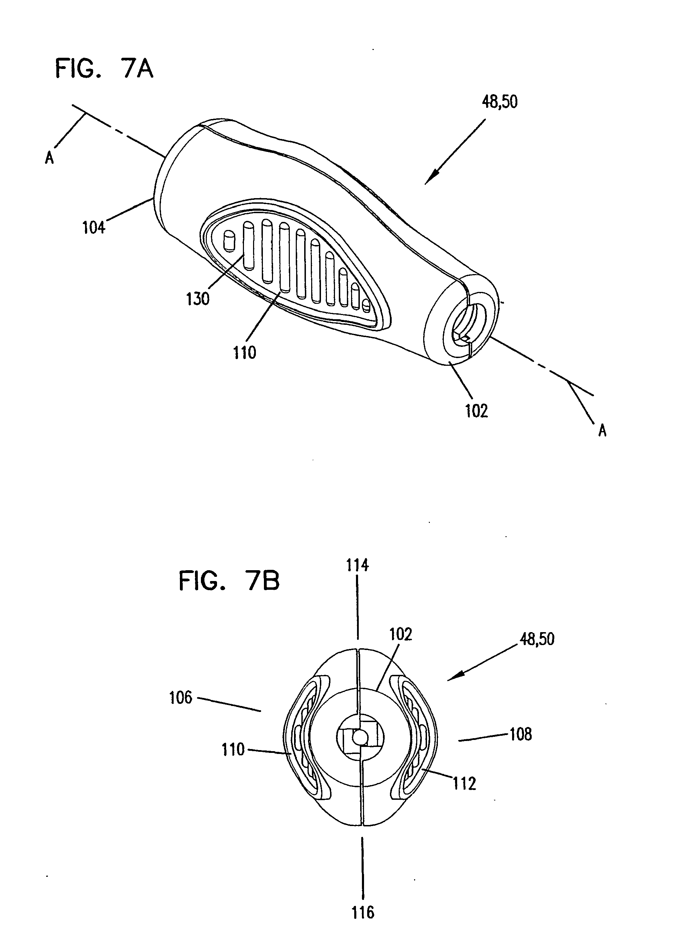

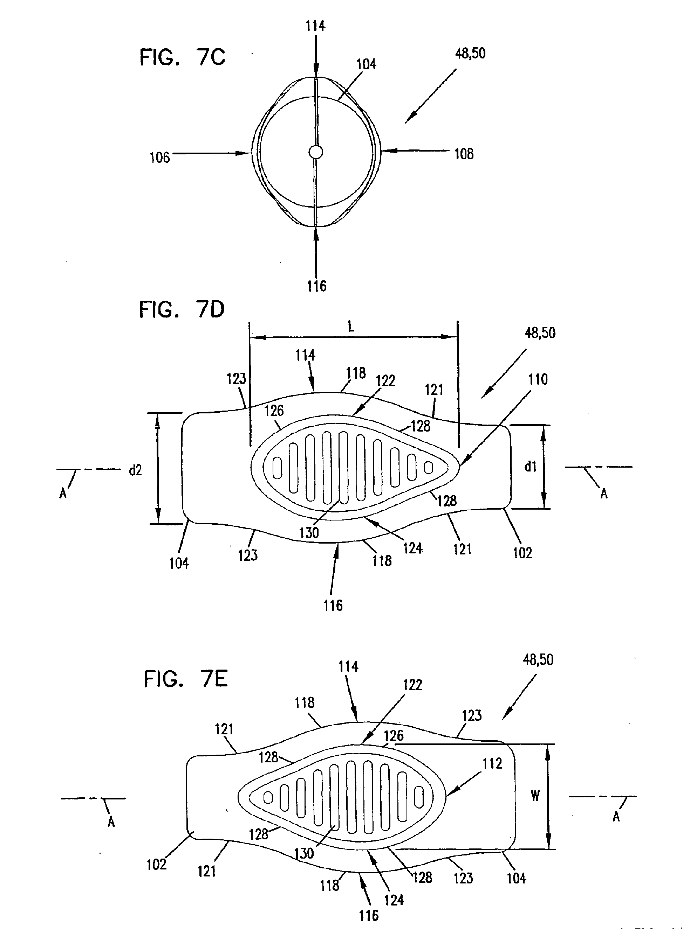

Figs. 7A-7G show one of the handles 48, 50 in isolation from the delivery system 10. The depicted handle 48, 50 is elongated along a central axis A-A and includes a first end 102 positioned opposite from a second end 104. The first end 102 preferably has a smaller perimeter (i.e., circumference) than the second end 104. For example, as shown in Fig. 7D, the first end preferably has a radial dimension d1 (i.e., the diameter of the first end 102) that is smaller than a radial dimension d2 of the second end 104 (i.e., the diameter of the second end 104). Preferably, the ends 102 and 104 have a generally round perimeter.

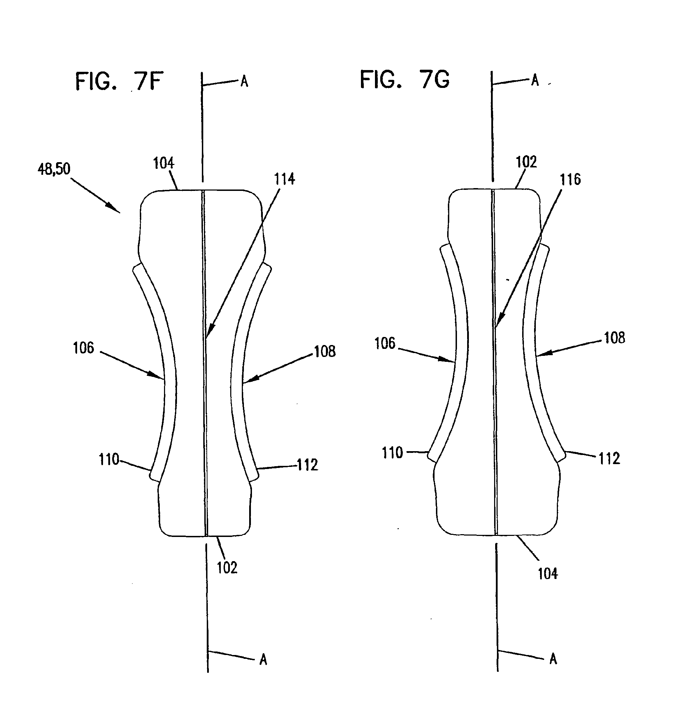

Referring to Figs. 7F and 7G, the handle 48, 50 also includes first and second sides 106 and 108 that extend longitudinally between the first and second ends 102 and 104. The first and second sides 106 and 108 preferably face in opposite directions. Concave gripping regions 110 and 112 are located at the first and second sides 106 and 108. The concave gripping regions 110 and 112 each define a concave curvature as the gripping regions 110, 112 extend in a longitudinal direction (i.e., along axis A-A) between the first and second ends 102 and 104.

Referring to Figs 7D and 7E, the handle 48, 50 also includes third and fourth sides 114 and 116 that extend longitudinally between the first and second ends 102 and 104. The third and fourth sides 114 and 116 face in opposite directions, and extend circumferentially (about the axis A-A) between the first and second sides 106 and 106. Preferably, the third and fourth sides 114 and 116 include convex regions 118 that extend in a longitudinal direction along an intermediate region of the handle 48, 50, and concave regions 121 and 123 that extend from the convex regions to the ends 102 and 104 of the handle 48, 50. The third and fourth sides 114 and 116 also define a convex curvature that extends in a circumferential direction (i.e., about the axis A-A as best shown in Figs. 7B and 7C).

Referring again to Figs. 7D and 7E, a length L of the concave gripping regions 110, 112 is preferably shorter than a total length of the handle 48, 50. Also, the gripping regions 110, 112 are preferably generally centered along the total length of the handle 48, 50. Additionally, the regions 110, 112 preferably include top and bottom edges 122 and 124 having convex curvatures 126 that transition into concave curvatures 128 adjacent the first end 102. The regions 110, 112 preferably have a maximum transverse width W at an intermediate position along their lengths L. The width W is preferably measured in a direction transverse relative to the axis A-A. The regions 110, 112 also preferably include elongated gripping projections 130. The gripping projections 130 are preferably parallel to one another, and preferably extend in a transverse direction relative to the axis A-A. The projections 130 are preferably longer at the intermediate positions of the gripping regions 110, 112 than adjacent the ends of the gripping regions 110, 112. In one non-limiting embodiment, the main body of the handle 48, 50 is made of a relatively hard material (e.g., polybutylene terephthalate) and the gripping regions 110, 112 are made of a softer, more resilient material (e.g., an overmolded polyester elastomer).

In an alternative embodiment and in accord with the principles of the first embodiment, the stent delivery system may further relate to a stent delivery system concerning balloon expandable stents. Also, the principles may be used in a balloon catheter system that may or may not have stent delivery capabilities.

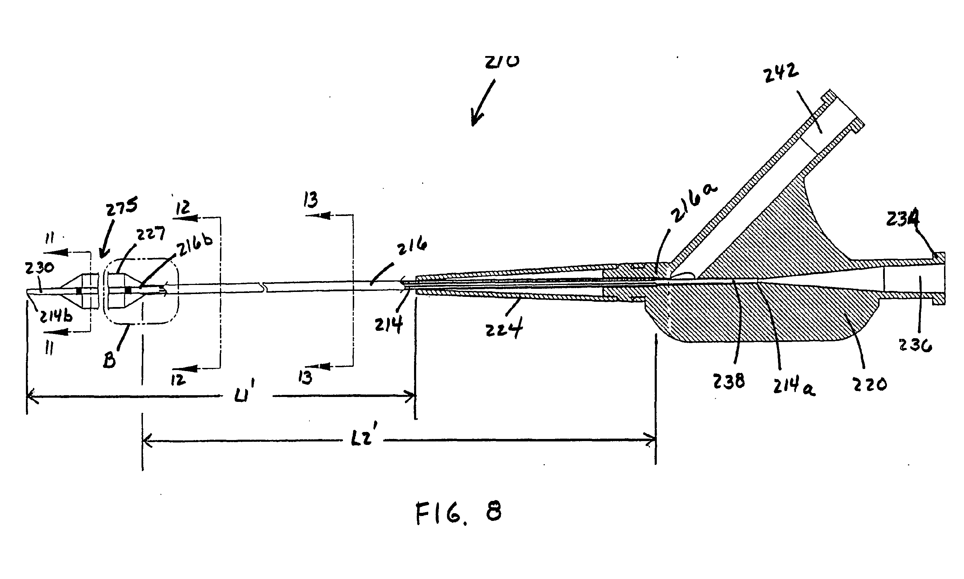

Referring now to Fig. 8, a second embodiment of the stent delivery system 210 providing for delivery of stents is shown having a manifold housing 220, an admission or fluid port 242, a guide wire port 234 having a tapered bore 236, and a strain relief jacket 224.

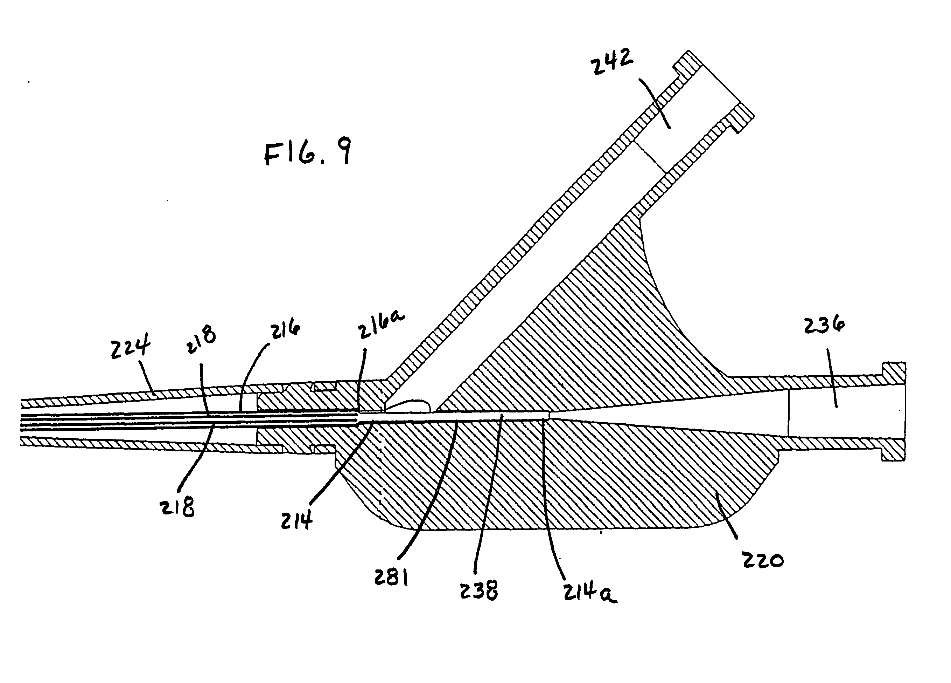

Similar to the preceding embodiment, the stent delivery system 210 includes an inner tubular member 214 and an outer tubular member 216. Referring to Fig. 8, each tubular member has proximal ends 214a and 216a and distal ends 214b and 216b. As shown in Figs. 12 and 13, a first lumen or fluid channel 240 is defined between the inner and outer tubular members 214 and 216. As shown in Fig. 9, the proximal end 214a of the inner tubular member passes through the strain relief jacket 224 and into the manifold housing 220. The inner tubular member 214 may be adhesively secured to the manifold housing 220 along a bonded area 281. The tapered bore 236 is aligned with an inner lumen 238 of the tubular member 214. The inner lumen 238 extends completely through the inner tubular member 214 so that the entire delivery system 210 can be passed over a guide wire (not shown) initially positioned within the patient's lumen.

The outer tubular member 216 defines a usable or operating length L1' of the stent delivery system. The operating length L1' includes a portion of the stent delivery system that is inserted into a patient's lumen. The operating length L1' extends from the strain relief jacket 224 to the end of a distal tip member 230, as shown in Fig. 8. The operating length may comprise a variety of lengths, including: 60 cm, 80 cm, 120 cm, 135 cm, and 150 cm.

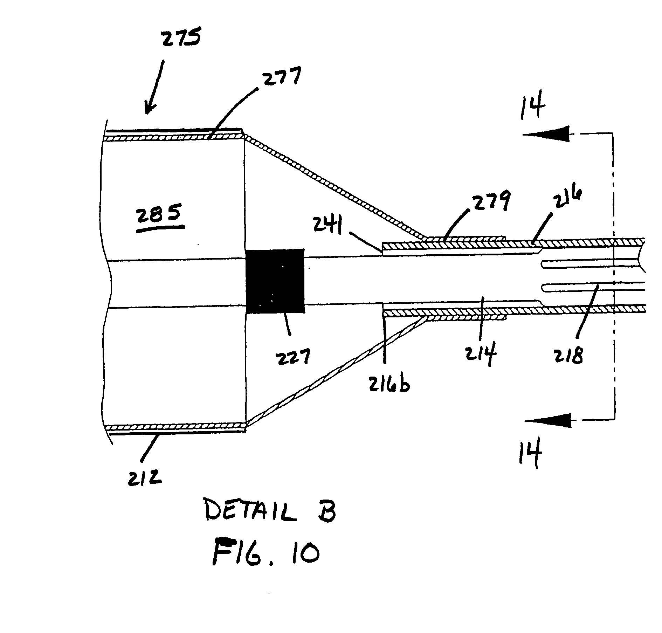

The fluid channel 240 has a fluid channel length L2', shown generally in Fig. 8. The fluid channel length L2 extends from the proximal end of the outer tubular member 216a, shown in Fig. 9, to the distal end of the outer tubular member 216b, shown in Fig 10. The spacer member 218 (shown in greater detail in Figs. 12-24) traverses along a predetermined percentage of the fluid channel length L2'. The predetermined percentage may be at least 10%, at least 25%, at least 50%, or at least 75% of the fluid channel length L2'. Preferably, the predetermined percentage over which the spacer member 218 traverses the fluid channel length L2 is at least 90%. Similarly, the spacer member 218 may traverse along a predetermined percentage of the operating length L1'. In certain embodiments, the spacer member 218 may extend into the balloon cavity and be longer than the fluid channel 240.

The distal end of the outer tubular member 216b is connected to a stent deployment arrangement 275 (see Figs. 8 and 10). The stent deployment arrangement 275 includes a balloon 277 (shown expanded in Fig. 8, 10 and 11) which defines an interior portion 285. The distal end of the inner tubular member 214b extends through the interior portion 285 of the balloon 277. A discharge port 241 located at the distal end of the outer tubular member 216b provides fluid communication between the fluid channel 240 and the interior portion 285 of the balloon 277.

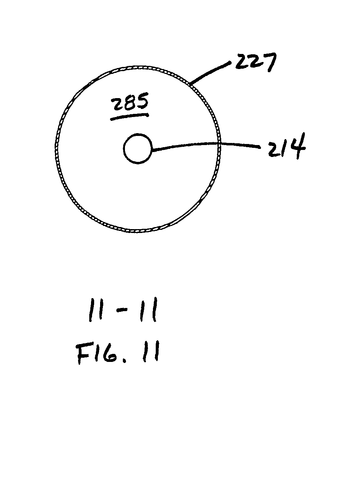

Fig. 11 depicts a cross section of the stent deployment arrangement 275 of Fig. 8 taken along the line 11-11. As shown in Fig. 11, the balloon 277 may comprise a circular cross section circumscribing the interior portion 285 through which the inner tubular member 214 extends. The balloon may further have a triangular or square shape, or any other shape advantageous for use (e.g., other shapes that may facilitate folding of the balloon).

In operation, a stent 212 is compressed about the inner tubular member 214 and the balloon 277 while the balloon is deflated. As so compressed, the stent 212 has a reduced diameter that permits the stent to be passed through the patient's vasculature to a deployment site. Once the system 210 has delivered the stent 212 to the deployment site, fluid is injected into the fluid port 242 and transferred through the fluid channel 240 and into the balloon 277. In response, the balloon expands thereby deforming the stent beyond its elastic limit to a permanently expanded form. After such expansion, the stent delivery system can be proximally withdrawn through the expanded stent and removed.

Referring again to Fig. 10, the balloon 277 may be an integral construction of the outer tubular member 216 or constructed by securely joining a connecting portion 279 of the balloon 277 to the outer tubular member 216. The connecting portion 279 may be joined to the outer tubular member 216 by, for example, common welding techniques or reflowing material processes.

Figs. 12 and 13 are cross sections of the stent delivery system 210 of Fig. 8, taken along lines 12-12 and 13-13, respectively. These illustrations show the inner and outer tubular members 214 and 216 and one embodiment of spacer members 218. In comparing the cross sections, the tubular members are preferably continuously and uniformly spaced along their length by the spacer members 218. This configuration can be used in both embodiments of the stent delivery system 10, 210. The spacer members 18, 218 maintain a predetermined spacing between the inner and outer tubular members 14, 214 and 16, 216 to maintain a uniform cross-sectional area of the channel 40, 240 within the length of the inner and outer tubular members through which, for example, fluid may flow. The fluid channel 240 in a balloon expandable stent delivery embodiment extends from the proximal end towards the distal end to provide fluid communication from the fluid port 242 through the distal opening 241 and to the balloon 277 for stent expansion. In similar fashion, the channel 40 in a self-expanding stent delivery embodiment extends from the proximal end towards the distal end to permit fluid flow to the discharge ports 41.

Generally, the spacer members 18, 218 comprise splines that radially project and extend substantially the entire axial length of the tubular members between the proximal end 16b, 216b of the outer tubular member 16, 216 and the proximal radio-opaque marker 27, 227. With respect to each spacer member embodiment, the radial dimension and axial length of each of the splines is identical and, in preferred embodiments, have a continuous uninterrupted length. However, it will be appreciated that the radial dimensions need not be identical. Further the splines need not have an uninterrupted length. Rather the splines may include interrupted lengths that start and stop at predetermined locations. The splines 18, 218 as illustrated, are examples of spacer member embodiments used to maintain a space between the outer tubular member 16, 216 and inner tubular member 14, 214.

Typically, the spacer members 18, 218 keep the inner tubular members 14, 214 in concentric alignment with their respective outer tubular member 16, 216. This permits the use of a small diameter inner tubular member 14, 214 relative to the diameter of the outer tubular member 16, 216 to increase the cross-sectional area of the first lumen 40, 240. Increasing the cross-sectional area of the first lumen 40, 240 reduces any impediment to flow of contrast media or fluid through the first lumen 40, 240 and increases the volume of contrast media or fluid within the first lumen 40, 240.

The spacers 18, 218 also resist kinking of the outer tubular members 16, 216 by providing structural reinforcement. The structural reinforcement thereby assists in preventing the channel 40, 240 from being constricted as the delivery system is flexed or bent through a patient's vasculature. Similarly, the spacers 18, 218 provide structural reinforcement to resist or eliminate crushing or compression of the outer tubular member against the inner tubular member, which also constricts the channel as the delivery system is positioned. A further advantageous feature of the spacers is that the spacers 18, 218 reduce or prevent inadvertent axial movement between the outer tubular member and the inner tubular member. For example, in an arrangement without spacers, the inner tubular member may bow or bend within the outer tubular member. Repeated areas of bending and bowing allow the inner tubular member to "snake" within or axially move relative to the outer tubular member. The spacer 18, 218 restricts bowing or inadvertent axial movement of the inner tubular member.

Referring again to Figs. 12 and 13, the spacer members 218 may be configured such that the spacer members 218 are constructed as an integral member of only one of the tubular members, the inner tubular member 214 for example. It will be appreciated that the spacer members may be integral with either or both tubular members. Fig. 14 (which is a cross section of the distal end of the stent delivery system shown in Fig. 10) discloses that the spacer members 218 may include a bonding surface 283 that may be bonded to provide fixed contact between both the inner tubular member 214 and the outer tubular member 216 of the balloon stent delivery system 210. The bonding surface 283 may be joined to a tubular member by, for example, a thermal bonding process or an adhesive. The bonding surface 283 may, as illustrated, bond to the inner surface of the outer tubular member 216, or in the alternative, bond to the outer surface of the inner tubular member, in which case the spacer member extends from the outer tubular member. The bonding surface resists or prevents axial movement between the inner and outer tubular members. Bonding surfaces 283 may be located along any location of the spacer member 218, or along the entire length of the spacer member 218. Preferably, the bonding surfaces 283 are located proximate the distal end of the outer tubular member 216b.

It is to be understood that spacer members depicted in the self-expanding stent delivery system and the balloon dilation stent delivery system, may comprise a variety of cross sectional configurations. It will further be appreciated that the radial dimensions need not be identical and the spline configurations of the spacer members need not have an uninterrupted length. Exemplary cross sections of various embodiments of the spacer members are shown in Figs. 15-23. The configurations are applicable to both the balloon expandable and self-expandable stent delivery systems described above. As is depicted, the spacer members may include a single spacer member or a plurality of spacer members.

Fig. 15 discloses a cross sectional configuration of a third embodiment of the present invention having an outer tubular member 216c, an inner tubular member 214c, and spacer members 218c with rounded ends. The inner tubular member 214c has an inner lumen 238c and the inner and outer tubular members 214c and 216c define a channel 240c. This configuration comprises five spacer members 218c integral with the inner tubular member 214c, each spacer member extending toward and contacting the outer tubular member 216c.

Fig. 16 discloses a cross sectional configuration of a fourth embodiment of the present invention, similar to that in Fig. 15, having an outer tubular member 216d, an inner tubular member 214d, and spacer members 218d with rounded ends. In this embodiment, eight spacer members 218d integral with the inner tubular member 214d are illustrated, each spacer member extending toward and contacting the outer tubular member 216d.

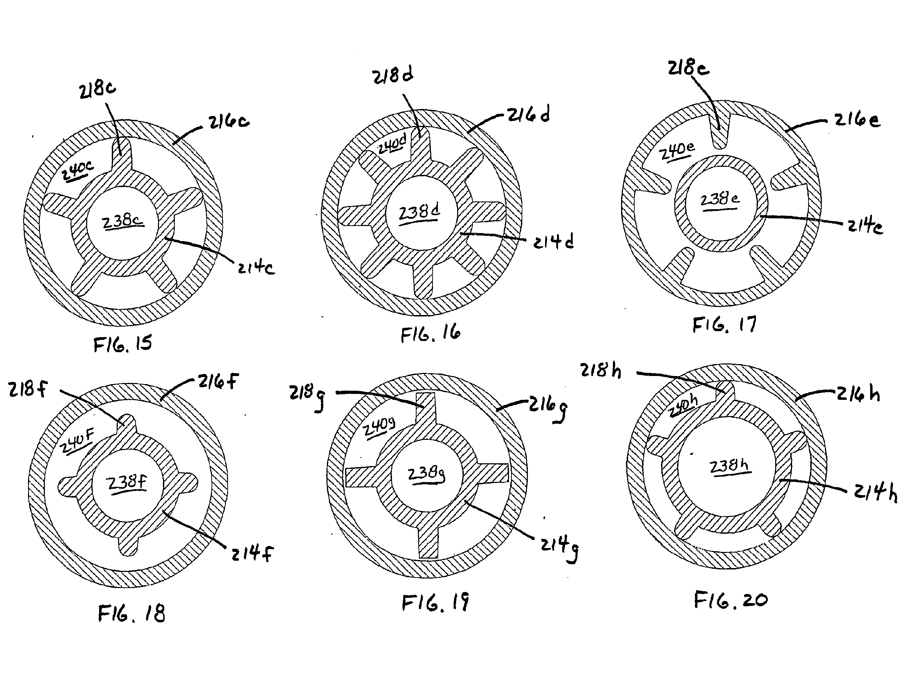

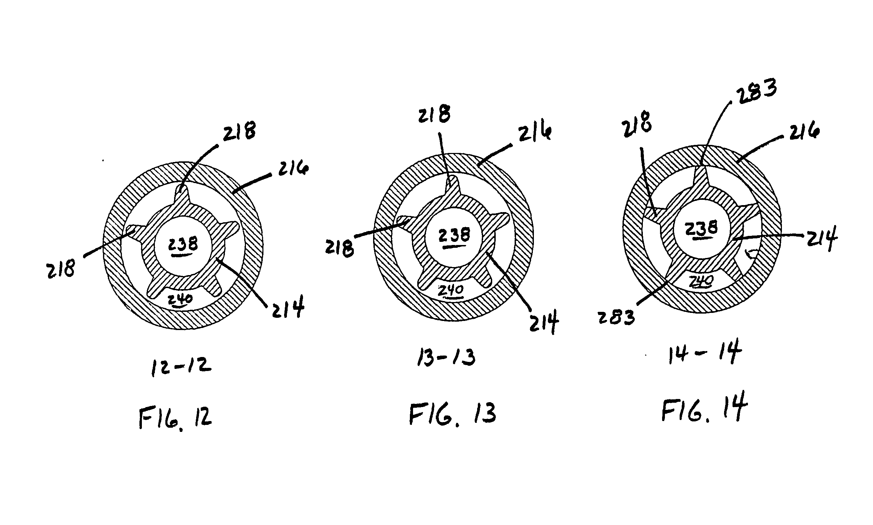

Fig. 17 discloses a cross sectional configuration of a fifth embodiment of the present invention having an outer tubular member 216e, an inner tubular member 214e, and spacer members 218e. The spacer members 218e of this embodiment discloses a conical cross section shape. The inner tubular member 214e has an inner lumen 238e and the inner and outer tubular members 214e and 216e define a channel 240e. Five spacer members 218e integral with the outer tubular member 216e are illustrated, each spacer member extending inward toward the inner tubular member 216e.

Fig. 18 discloses a cross sectional configuration of a sixth embodiment of the present invention, having an outer tubular member 216f, an inner tubular member 214f, and shorter spacer members 218f with rounded ends. In this embodiment, four shorter spacer members 218f integral with the inner tubular member 214f are illustrated, each spacer member extending toward the outer tubular member 216d, but not contacting the outer tubular member 216d.

Fig. 19 discloses a cross sectional configuration of a seventh embodiment of the present invention, having an outer tubular member 216g, an inner tubular member 214g, and spacer members 218g with squared ends. In this embodiment, four spacer members 218g integral with the inner tubular member 214g are illustrated, each spacer member extending toward the outer tubular member 216f. As illustrated the square spacer members 218g do not contact the outer tubular member 216g, but may contact the outer tubular member in alternative embodiments.

Fig. 20 discloses a cross sectional configuration of an eighth embodiment of the present invention, having an outer tubular member 216h, an inner tubular member 214h, and shorter spacer members 218h with rounded ends. In this embodiment, the inner lumen 23 8h of the inner tubular member 214h has a larger diameter than other embodiments previously illustrated. It is contemplated that in alternative embodiments, the inner lumen diameter may be smaller than the diameter of other embodiments illustrated. Four shorter spacer members 218h integral with the inner tubular member 214h are illustrated, each spacer member extending toward and contacting the outer tubular member 216h.

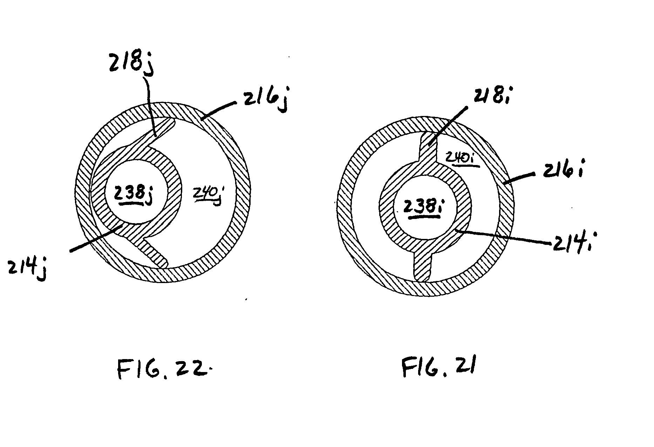

Fig. 21 discloses a cross sectional configuration of a ninth embodiment of the present invention, having an outer tubular member 216i, an inner tubular member 214i, and spacer members 218i with rounded ends. In this embodiment, two opposing spacer members 218i integral with the inner tubular member 214i are illustrated, each spacer member extending toward and contacting the outer tubular member 216i.

Fig. 22 discloses a cross sectional configuration of a tenth embodiment of the present invention, having an outer tubular member 216j, an inner tubular member 214j, and spacer members 218j. The spacer member configuration of this embodiment has an asymmetrical cross section wherein spacer members 218j of the inner tubular member 214j offset the inner tubular member against the inside wall of the outer tubular member 216j. It will further be appreciated that a spacer member on the outer tubular member may offset the inner tubular member against the inside wall of the outer tubular member.

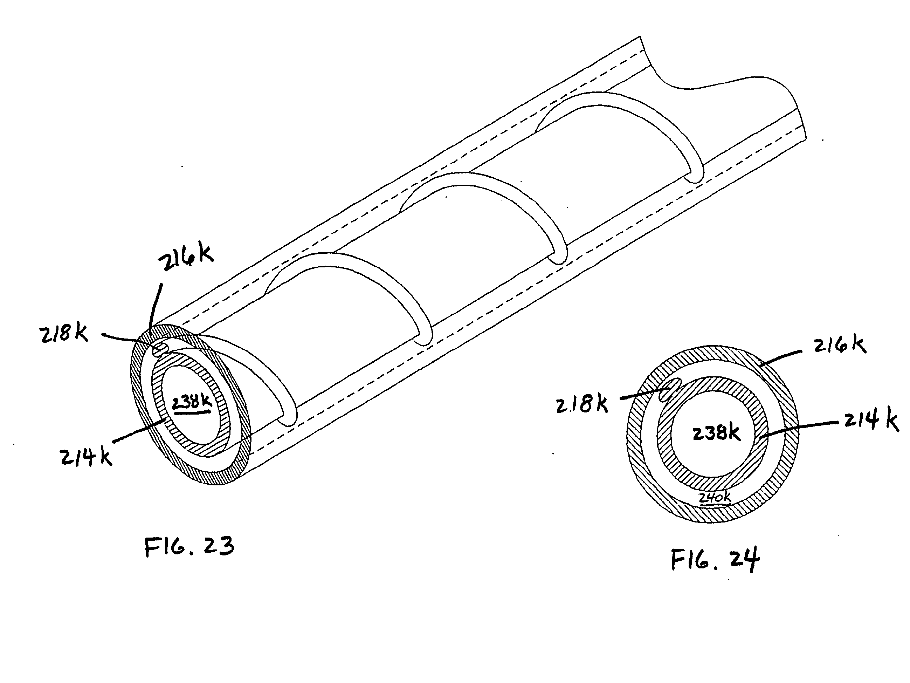

The spacer member configuration may also include non-spline spacer members. Figs. 23 and 24 disclose a cross sectional configuration of an eleventh embodiment of the present invention, having an outer tubular member 216k, an inner tubular member 214k, and helical spacer members 218k. The helical spacer member 218k is coiled around the inner tubular member 214k. Alternatively, the helical spacer member 218k may be integral to the inner diameter of the outer tubular member 216k. Other helical configurations, such as a plurality of helical spacer members, are contemplated.

As shown in the embodiments, the spacer member may be integral or joined to either the inner tubular member or the outer tubular member. It is further contemplated that a separate and independent spacer member may be provided within the fluid channel of the stent delivery system, or that both the inner and outer tubular members comprise integral spacer members.

It has been shown how the objects of the invention have been attained in a preferred manner.

## Claims

1. In a catheter system comprising:
an elongated, flexible, hollow outer tubular member having a distal end and a proximal end;
an elongated, flexible, inner tubular member having a distal end and a proximal end;
said inner tubular member disposed within said outer tubular member such that a fluid channel having a fluid channel length is defined between the inner and outer tubular members; and
a stent mounting location located near said distal ends of said inner and outer tubular members;
at least one spacer disposed within said fluid channel between said inner tubular member and said outer tubular member for maintaining a spacing between said inner tubular member and said outer tubular member, and
an admission port in fluid communication with said fluid channel
**characterized by**:
said spacer longitudinally traversing at least 10 percent of said fluid channel length.

2. The catheter system according to claim 1, wherein said spacer is a longitudinal spacer extending a majority of a length from said proximal end to said distal end of said inner and outer tubular members.

3. The catheter system according to claim 1, wherein said spacer is a continuous longitudinal extension traversing a majority of a length from said proximal end to said distal end of said inner and outer tubular members.

4. The catheter system according to claim 1, wherein said spacer traverses at least 25 percent of said fluid channel length.

5. The catheter system according to claim 1, wherein said spacer traverses at least 50 percent of said fluid channel length.

6. The catheter system according to claim 1, wherein said spacer traverses at least 75 percent of said fluid channel length.

7. The catheter system according to claim 1, wherein said spacer traverses a majority of said fluid channel length.

8. The catheter system according to claim 1, wherein said spacer is disposed to centrally position said inner tubular member within said outer tubular member.

9. The catheter system according to claim 1, wherein said spacer is disposed to maintain said inner tubular member in an offset position within said outer tubular member.

10. The catheter system according to claim 1, wherein said spacer is a spline elongated in a direction along a length of the catheter system.

11. The catheter system according to claim 10, wherein said catheter system includes a plurality of splines elongated along the length of the catheter system.

12. The catheter system according to claim 11, wherein said splines couple to said outer tubular member and project inwardly towards said inner tubular member.

13. The catheter system according to claim 11, wherein said splines couple to said inner tubular member and project outwardly towards said outer tubular member.

14. The catheter system according to claim 1, wherein said spacer includes a plurality of radial, spaced-apart spacer members that extend longitudinally along said fluid channel.

15. The catheter system according to claim 1, wherein said spacer comprises at least one helical spacer extending along a length of said fluid channel.

16. The catheter system according to claim 15, wherein said helical spacer is coupled to said inner tubular member and projects radially outward from said inner tubular member.

17. The catheter system according to claim 1, wherein said spacer includes at least one thermal bonding surface to fixedly couple said inner tubular member and said outer tubular member.

18. The catheter system according to claim 17, wherein said bonding surface is located adjacent the distal end of said outer tubular member.

19. The catheter system according to claim 1, wherein said inner tubular member is hollow to track over a guide wire.

20. The catheter system according to claim 1, including a discharge opening in fluid communication with said fluid channel, the discharge opening being located near said distal end of said outer tubular member.

21. The catheter system according to claim 20, wherein said discharge opening is formed in said outer tubular member to permit fluid flow from said fluid channel to a patient's lumen.

22. The catheter system according to claim 1, wherein said stent mounting location comprises a balloon arrangement for balloon stent delivery, said balloon arrangement being in fluid communication with said fluid channel.

23. The catheter system according to claim 1, wherein said stent mounting location comprises a self-expanding stent arrangement for self-expanding stent delivery, said stent being exposed by axially retracting said outer tubular member relative to said inner tubular member.

24. The catheter system of claim 1, further comprising an expandable balloon arrangement located near said distal end of said inner and outer tubular members, said expandable balloon arrangement being in fluid communication with said fluid channel.

## Patentansprüche

1. Kathetersystem mit:
einem länglichen, flexiblen, hohlen äußeren rohrförmigen Teil mit einem distalen Ende und einem proximalen Ende;
einem länglichen, flexiblen inneren rohrförmigen Teil mit einem distalen Ende und einem proximalen Ende;
wobei das innere rohrförmige Teil derart in dem äußeren rohrförmigen Teil angeordnet ist, dass zwischen dem inneren rohrförmigen Teil und dem äußeren rohrförmigen Teil ein Fluidkanal mit einer Fluidkanal-Länge ausgebildet ist; und
einer Stent-Befestigungsstelle, die nahe den distalen Enden des inneren rohrförmigen Teils und des äußeren rohrförmigen Teils angeordnet ist;
mindestens einem Abstandhalter, der in dem Fluidkanal zwischen dem inneren rohrförmigen Teil und dem äußeren rohrförmigen Teil angeordnet ist, um einen Abstand zwischen dem inneren rohrförmigen Teil und dem äußeren rohrförmigen Teil aufrechtzuerhalten, und
einem Einlassport, der sich in Fluidverbindung mit dem Fluidkanal befindet,
**dadurch gekennzeichnet,**
**dass** der Abstandhalter in Längsrichtung mindestens 10 Prozent der Länge des Fluidkanals einnimmt.

2. Kathetersystem nach Anspruch 1, bei dem der Abstandhalter ein längsverlaufender Abstandhalter ist, der sich entlang eines Großteils der Länge von dem proximalen Ende zu dem distalen Ende der inneren und äußeren rohrförmigen Teile erstreckt.

3. Kathetersystem nach Anspruch 1, bei dem der Abstandhalter ein durchgehendes längsverlaufendes Verlängerungsteil ist, das sich entlang eines Großteils der Länge von dem proximalen Ende zu dem distalen Ende der inneren und äußeren rohrförmigen Teile erstreckt.

4. Kathetersystem nach Anspruch 1, bei dem der Abstandhalter mindestens 25 Prozent der Länge des Fluidkanals einnimmt.

5. Kathetersystem nach Anspruch 1, bei dem der Abstandhalter mindestens 50 Prozent der Länge des Fluidkanals einnimmt.

6. Kathetersystem nach Anspruch 1, bei dem der Abstandhalter mindestens 75 Prozent der Länge des Fluidkanals einnimmt.

7. Kathetersystem nach Anspruch 1, bei dem sich der Abstandhalter entlang eines Großteils der Länge des Fluidkanals erstreckt.

8. Kathetersystem nach Anspruch 1, bei dem der Abstandhalter derart angeordnet ist, dass er das innere rohrförmige Teil mittig in dem äußeren rohrförmigen Teil positioniert.

9. Kathetersystem nach Anspruch 1, bei dem der Abstandhalter derart angeordnet ist, dass er das innere rohrförmige Teil in einer versetzten Position in dem äußeren rohrförmigen Teil hält.

10. Kathetersystem nach Anspruch 1, bei dem der Abstandhalter ein Keil ist, dessen Längserstreckung in einer Richtung entlang der Länge des Kathetersystems verläuft.

11. Kathetersystem nach Anspruch 10, wobei das Kathetersystem mehrere entlang der Länge des Kathetersystems langgestreckte Keile aufweist.

12. Kathetersystem nach Anspruch 11, bei dem die Keile mit dem äußeren rohrförmigen Teil verbunden sind und nach innen zu dem inneren rohrförmigen Teil hin abstehen.

13. Kathetersystem nach Anspruch 11, bei dem die Kelle mit dem inneren rohrförmigen Teil verbunden sind und nach außen zu dem äußeren rohrförmigen Teil hin abstehen.

14. Kathetersystem nach Anspruch 1, bei dem der Abstandhalter mehrere radiale, voneinander beabstandete Abstandhalterteile aufweist, die sich in Längsrichtung entlang des Fluidkanals erstrecken.

15. Kathetersystem nach Anspruch 1, bei dem der Abstandhalter mindestens einen schraubenlinienförmigen Abstandhalter aufweist, des sich entlang eines Längenbereichs des Fluidkanals erstreckt.

16. Kathetersystem nach Anspruch 15, bei dem der schraubenlinienförmige Abstandhalter mit dem inneren rohrförmigen Teil verbunden ist und nach außen von dem inneren rohrförmigen Teil absteht.

17. Kathetersystem nach Anspruch 1, bei dem der Abstandhalter mindestens eine Wärmebondungsfläche aufweist, um das innere rohrförmige Teil und das äußere rohrförmige Teil fest miteinander zu verbinden.

18. Kathetersystem nach Anspruch 17, bei dem die Bondungsfläche nahe dem distalen Ende des äußeren rohrförmigen Teils angeordnet ist.

19. Kathetersystem nach Anspruch 1, bei dem das innere rohrförmige Teil hohl ist, um über einen Führungsdraht geführt zu werden.

20. Kathetersystem nach Anspruch 1, mit einer Ausgabeöffnung, die sich in Fluidverbindung mit dem Fluidkanal befindet, wobei die Ausgabeöffnung nahe dem distalen Ende des äußeren rohrförmigen Teils angeordnet ist.

21. Kathetersystem nach Anspruch 20, bei dem die Ausgabeöffnung in dem äußeren rohrförmigen Teil ausgebildet ist, um einen Fluidstrom aus dem Fluidkanal in ein Lumen des Patienten zu ermöglichen.

22. Kathetersystem nach Anspruch 1, bei dem die Stent-Befestigungsstelle eine Ballonvorrichtung zur Ballon-Stentzufuhr aufweist, wobei die Ballonvorrichtung sich in Fluidverbindung mit dem Fluidkanal befindet.

23. Kathetersystem nach Anspruch 1, bei dem die Stent-Befestigungsstelle eine selbstaufweitende Stent-Anordnung zur Zufuhr mit selbstaufweitendem Stent aufweist, wobei der Stent freigelegt wird, indem das äu-Bere rohrförmige Teil relativ zu dem inneren rohrförmigen Teil axial zurückgezogen wird.

24. Kathetersystem nach Anspruch 1, ferner mit einer aufweitbaren Ballonvorrichtung, die nahe dem distalen Ende der inneren und äußeren rohrförmigen Teile angeordnet ist, wobei die aufweitbare Ballonvorrichtung sich in Fluidverbindung mit dem Fluidkanal befindet.

## Revendications

1. Système de cathéter comprenant :
un élément tubulaire externe creux, flexible et allongé présentant une extrémité distale et une extrémité proximale ;
un élément tubulaire interne flexible et allongé présentant une extrémité distale et une extrémité proximale ;
ledit élément tubulaire interne disposé à l'intérieur dudit élément tubulaire externe de telle sorte qu'un canal fluidique présentant une longueur de canal fluidique est défini entre les éléments tubulaires interne et externe ; et
un site de montage de stent situé à proximité desdites extrémités distales desdits éléments tubulaires interne et externe ;
au moins une pièce d'espacement disposée à l'intérieur dudit canal fluidique entre ledit élément tubulaire interne et ledit élément tubulaire externe pour maintenir un espacement entre ledit élément tubulaire interne et ledit élément tubulaire externe, et
un orifice d'admission en communication fluidique avec ledit canal fluidique **caractérisé par** :
ladite pièce d'espacement traversant de manière longitudinale au moins 10 pour cent de ladite longueur du canal fluidique.

2. Système de cathéter selon la revendication 1, dans lequel ladite pièce d'espacement est une pièce d'espacement longitudinale s'étendant sur une majorité d'une longueur allant de ladite extrémité proximale à ladite extrémité distale desdits éléments tubulaires interne et externe.

3. Système de cathéter selon la revendication 1, dans lequel ladite pièce d'espacement est une extension longitudinale continue traversant une majorité d'une longueur allant de ladite extrémité proximale à ladite extrémité distale desdits éléments tubulaires interne et externe.

4. Système de cathéter selon la revendication 1, dans lequel ladite pièce d'espacement traverse au moins 25 pour cent de ladite longueur du canal fluidique.

5. Système de cathéter selon la revendication 1, dans lequel ladite pièce d'espacement traverse au moins 50 pour cent de ladite longueur du canal fluidique.

6. Système de cathéter selon la revendication 1, dans lequel ladite pièce d'espacement traverse au moins 75 pour cent de ladite longueur du canal fluidique.

7. Système de cathéter selon la revendication 1, dans lequel ladite pièce d'espacement traverse une majorité de ladite longueur du canal fluidique.

8. Système de cathéter selon la revendication 1, dans lequel ladite pièce d'espacement est disposée de façon à positionner de manière centrale ledit élément tubulaire interne à l'intérieur dudit élément tubulaire externe.

9. Système de cathéter selon la revendication 1, dans lequel ladite pièce d'espacement est disposée de façon à maintenir ledit élément tubulaire interne dans une position décalée à l'intérieur dudit élément tubulaire externe.

10. Système de cathéter selon la revendication 1, dans lequel ladite pièce d'espacement est une cannelure allongée dans un sens le long d'une longueur du système de cathéter.

11. Système de cathéter selon la revendication 10, dans lequel ledit système de cathéter comprend une pluralité de cannelures allongées le long de la longueur du système de cathéter.

12. Système de cathéter selon la revendication 11, dans lequel lesdites cannelures s'accouplent au dit élément tubulaire externe et se projettent vers l'intérieur vers l'élément tubulaire interne.

13. Système de cathéter selon la revendication 11, dans lequel lesdites cannelures s'accouplent au dit élément tubulaire interne et se projettent vers l'extérieur vers l'élément tubulaire externe.

14. Système de cathéter selon la revendication 1, dans lequel ladite pièce d'espacement comprend une pluralité d'éléments d'espacement radiaux disposés à distance les uns des autres qui s'étendent de manière longitudinale le long dudit canal fluidique.

15. Système de cathéter selon la revendication 1, dans lequel ladite pièce d'espacement comprend au moins une pièce d'espacement hélicoïdale s'étendant le long d'une longueur dudit canal fluidique.

16. Système de cathéter selon la revendication 15, dans lequel ladite pièce d'espacement hélicoïdale est couplée au dit élément tubulaire interne et se projette radialement vers l'extérieur depuis ledit élément tubulaire interne.

17. Système de cathéter selon la revendication 1, dans lequel ladite pièce d'espacement comprend au moins une surface thermocollante pour coupler solidement ledit élément tubulaire interne et ledit élément tubulaire externe.

18. Système de cathéter selon la revendication 17, dans lequel la surface collante est située de manière adjacente à l'extrémité distale dudit élément tubulaire externe.

19. Système de cathéter selon la revendication 1, dans lequel élément tubulaire interne est creux pour recevoir un fil guide.

20. Système de cathéter selon la revendication 1, comprenant une ouverture d'évacuation en communication fluidique avec ledit canal fluidique, l'ouverture d'évacuation étant située à proximité de ladite extrémité distale dudit élément tubulaire externe.

21. Système de cathéter selon la revendication 20, dans lequel ladite ouverture d'évacuation est formée dans ledit élément tubulaire externe pour permettre au fluide de s'écouler dudit canal fluidique jusqu'à une lumière du patient.

22. Système de cathéter selon la revendication 1, dans lequel ledit site de montage de stent comprend un agencement formant ballonnet en vue du largage du stent à ballonnet, ledit agencement formant ballonnet étant en communication fluidique avec ledit canal fluidique.

23. Système de cathéter selon la revendication 1, dans lequel ledit site de montage de stent comprend un agencement formant stent auto-expansible en vue du largage du stent auto-expansible, ledit stent étant mis à nu en rétractant de manière axiale ledit élément tubulaire externe par rapport au dit élément tubulaire interne.

24. Système de cathéter selon la revendication 1, comprenant en outre un agencement formant ballonnet expansible situé à proximité de ladite extrémité distale desdits éléments tubulaires interne et externe, ledit agencement formant ballonnet expansible étant en communication fluidique avec ledit canal fluidique.
